# EUROPEAN PATENT APPLICATION

(11) **EP 3 501 395 A1**
(43) Date of publication of application: **26.06.2019**
(21) Application number: 17209004.5
(22) Date of filing: 20.12.2017
(51) Int. Cl.: A61B 5/1455

(54) **AN APPARATUS AND ASSOCIATED METHODS FOR SWEAT RECEPTION**

(71) Applicant: Nokia Technologies Oy, 02610 Espoo (FI)
(72) Inventor: Woldegebriel,, Michael, 02610 Espoo (FI)
(74) Representative: Potter Clarkson

(57) **Abstract**

A hand device module, the module comprising an attachment face and a sweat receptor face, the attachment face configured to be attached to a hand device and the sweat receptor face configured to receive sweat when the module is attached to the hand device, the module configured to provide the received sweat to a sweat detector, the sweat detector configured to detect one or more properties of the received sweat.

## Description

### Technical Field

The present disclosure relates to apparatus and methods associated with capturing, and in some cases analysing, a user's sweat.

Some examples may relate to portable electronic devices, in particular, so-called hand-portable electronic devices which may be hand-held in use (although they may be placed in a cradle in use). Such hand-portable electronic devices include so-called Personal Digital Assistants (PDAs) and tablet PCs. Certain portable electronic devices may be wearable, such as on the wrist. The portable electronic devices/apparatus according to one or more disclosed example aspects/embodiments may provide one or more audio/text/video communication functions (e.g. telecommunication, videocommunication, and/or text transmission, Short Message Service (SMS)/ Multimedia Message Service (MMS)/emailing functions, interactive/non-interactive viewing functions (e.g. web-browsing, navigation, TV/program viewing functions), music recording/playing functions (e.g. MP3 or other format and/or (FM/AM) radio broadcast recording/playing), downloading/sending of data functions, image capture function (e.g. using a (e.g. in-built) digital camera), and gaming functions.

### Background

Recent developments in technology include devices used to monitor a user's health and wellbeing.

The listing or discussion of a prior-published document or any background in this specification should not necessarily be taken as an acknowledgement that the document or background is part of the state of the art or is common general knowledge.

### Summary

According to a first aspect, there is provided a hand device module, the module comprising:
an attachment face and a sweat receptor face, the attachment face configured to be attached to a hand device and the sweat receptor face configured to receive sweat when the module is attached to the hand device, the module configured to provide the received sweat to a sweat detector, the sweat detector configured to detect one or more properties of the received sweat.

The module may be a hand portable electronic device module.

The sweat detector may be integrated with the module.

The sweat detector may be configured to detect the one or more properties of the sweat, wherein the one or more properties of the sweat comprises a refraction property of the sweat.

The sweat detector may be configured to detect one or more properties of the sweat by using one or more chemical compositions which interact with the sweat according to the one or more properties of the sweat.

The interaction of the one or more chemical compositions with the sweat may be a respective specific change in the colour of at least one of the one or more chemical compositions according to the detected one or more properties of the sweat.

The module may comprise one or more chambers. The one or more chemical compositions may be comprised in the one or more respective chambers of the module. The one or more chambers may be configured to provide access for an optical sensor configured to determine a refraction property of the sweat within the chamber

One or more of the chemical compositions (e.g. in respective chambers, or in the same chamber) may be configured to differentially interact with the sweat according to the one or more properties of the sweat.

The module may be configured to comprise one or more capillary channels configured to feed the received sweat from the sweat receptor face to the sweat detector. In examples in which one or more chambers are present in the module, the capillary channels may connect the exposed face of the module to the respective chambers.

The chemical compositions may comprise one or more of: cobalt chloride (CoCl₂); iodide (L); Hg²⁺ and Fe²⁺ with 2,4,6-tris(2-pyridiyl)-s-triazine (TPTZ); bromothymol blue; methyl red; and phenolphthalein.

The one or more properties of the received sweat may comprise an indication of the presence of one or more particular analyte compounds present in the received sweat, the one or more analyte compounds comprising: sodium (Na⁺); chloride (Cl⁻); potassium (K⁺); ammonium (NH₄⁺); ethanol; cortisol; urea; lactate; neuropeptides and cytokines.

The one or more properties of the received sweat of the user may comprise an indication of one or more medical/physiological conditions of the user, the medical/physiological conditions comprising one or more of: cystic fibrosis, dehydration, endocrine disease, diabetes, adrenal disease, thyroid disease, pituitary glands disease, blood pressure disorders, muscle contractions, nerve impulses, digestion disorders, heart rhythm problems, liver problems, kidney problems, electrolyte imbalance; intoxication /drunkenness; stress; high level of anaerobic activity, and depressive/personality disorders

The attachment face and the sweat receptor face may each be on opposing faces of the module.

The attachment face may be configured to be repeatably attached and detached to the hand device. The attachment face may be repeatably attached by one or more of an adhesive and a physical locking element.

The module may be configured to transfer the received sweat to a sweat detector integrated with the hand device.

In a further aspect there is provided a hand device and a hand device module, the module comprising an attachment face and a sweat receptor face, the attachment face configured to be attached to the hand device and the sweat receptor face configured to receive sweat when the module is attached to the hand device, the module configured to provide the received sweat to a sweat detector, the sweat detector configured to detect one or more properties of the received sweat. The hand device may thus be attachable to the attachment face of the hand device module, so that when the hand device is attached to the hand device module, the sweat receptor face of the hand device module faces outwardly towards a user of the hand device.

The module may comprise means for providing the received sweat from the sweat receptor face to the sweat detector, the means comprising one or more of: a capillary channel; a pore; a valve; an opening; and a sponge.

In a further aspect there is provided a hand device comprising a sweat receptor, the sweat receptor positioned for engagement with one or more of the palm(s) and digit(s) of a user's hand(s) when the hand portable electronic device is being held in one or more designed operative in-use holding positions, the sweat receptor configured to be operatively connected to a sweat detector of the hand device.

The hand device may be a hand portable electronic device.

The sweat detector may be configured to detect one or more of:
a refraction property of the sweat; and
one or more properties of the sweat by using one or more chemical compositions which interact with the sweat according to the one more properties of the sweat.

The hand device may comprise one or more chambers, configured to receive the sweat from the sweat receptor. The one or more chambers may comprise one or more chemical compositions configured to interact with the sweat according to the one or more properties of the received sweat. The one or more chambers may comprise access to an optical sensor configured to determine a refraction property of the sweat within the chamber.

The sweat detector may comprise one or more of:
one or more cameras configured to capture one or more changes in colour, due to interaction with the sweat, of the one or more chemical compositions in the one or more chambers; and
one or more optical sensors configured to capture a refraction property of the sweat.

The hand device may be configured to use the captured refraction property of the sweat to determine a refractive index of the sweat, the refractive index of the sweat indicative of one or more predefined concentrations of soluble compounds contained in the sweat.

The one or more cameras and optical sensors may be located at a hand device side of the sweat receptor opposite a sweat receptor side of the sweat receptor.

The sweat detector may be configured to detect a change in one of more of:
the detected refraction property of a chamber configured to receive sweat for refraction analysis; and
the colour of one or more chemical compositions which interact with the sweat according to the one more properties of the sweat, and
based on the detection of the change, trigger an optical sensor to capture the one or more of the detected refraction property and the colour of one or more chemical compositions for provision to an analysis module configured to providing a health status prediction to the user.

The sweat detector may be configured to provide the one of more of the detected refraction property of the sweat; and the one or more properties of the sweat by using one or more chemical compositions which interact with the sweat according to the one more properties of the sweat, to an analysis module configured to providing a health status prediction to the user.

The hand device may comprise the analysis module. The analysis module may be remote from and in communication with the hand device.

The hand device may be one or more of: a hand portable electronic device, a mobile telephone, a smartphone, a tablet computer, an electronic navigation device, a smartwatch, a music player, a case for a hand portable electronic device (e.g. a case for a smartphone, for a tablet computer, or for a music player), a writing implement, a steering wheel, a handlebar, a handle, a tool and a handheld computer peripheral.

In a further aspect there is provided a method comprising:
detecting, using a sweat detector, one or more properties of sweat received at a sweat receptor face of a hand device module, the hand device module comprising the sweat receptor face and an attachment face, the attachment face configured to be attached to a hand device and the sweat receptor face configured to receive sweat when the hand device module is attached to the hand device.

The steps of any method disclosed herein do not have to be performed in the exact order disclosed, unless explicitly stated or understood by the skilled person. The method may be a computer-implemented method.

Corresponding computer programs for implementing one or more steps of the methods disclosed herein are also within the present disclosure and are encompassed by one or more of the described examples.

In a further aspect there is provided a computer readable medium comprising computer program code stored thereon, the computer readable medium and computer program code being configured to, when run on at least one processor, perform: detecting, using a sweat detector, one or more properties of sweat received at a sweat receptor face of a hand device module, the hand device module comprising the sweat receptor face and an attachment face, the attachment face configured to be attached to a hand device and the sweat receptor face configured to receive sweat when the hand device module is attached to the hand device.

One or more of the computer programs may, when run on a computer, cause the computer to configure any apparatus, including a circuit, controller, or device disclosed herein, or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

One or more of the computer programs may be provided on a computer readable medium, which may be a physical computer readable medium such as a disc or a memory device, may be a non-transient medium, or may be embodied as a transient signal. Such a transient signal may be a network download, including an internet download.

The present disclosure includes one or more corresponding aspects, examples or features in isolation or in various combinations whether or not specifically stated (including claimed) in that combination or in isolation. Corresponding means for performing one or more of the discussed functions (e.g. attachment means, sweat detection means, sweat analysis means) are also within the present disclosure.

The above summary is intended to be merely exemplary and non-limiting.

### Brief Description of the Figures

A description is now given, by way of example only, with reference to the accompanying drawings, in which:
Figure 1a shows an example hand device, sweat detector and hand device module according to the present disclosure;
Figure 1b shows an example apparatus according to the present disclosure;
Figure 2 shows an example hand device and hand device module according to the present disclosure;
Figure 3 shows an example hand device module comprising a plurality of chemical compositions according to the present disclosure;
Figure 4 shows an example hand device module comprising a plurality of chambers containing respective chemical compositions according to the present disclosure;
Figure 5 shows an example chamber configured to allow the refraction property of sweat within the chamber to be determined according to the present disclosure;
Figure 6 shows an example method according to the present disclosure; and
Figure 7 shows a computer-readable medium comprising a computer program configured to perform, control or enable the method of Figure 6.

### Description of Specific Examples

Recent developments in technology include devices used to monitor a user's health and wellbeing, and help the user make lifestyle adjustments/improvements based on indications determined by such devices. In recent years, technological advancements allow for collection and analysis of minute amounts of sweat to make health related predictions (e.g. dehydration) using biomarkers present in the sweat.

It may be advantageous to be able to monitor the biomarkers present in a user's sweat without requiring the user to deliberately take action to provide their sweat for analysis. This may be termed "compliance-free monitoring" of the user's sweat. This may help improve the usefulness of such monitoring devices, since the user may benefit from sweat analysis without the burden of deliberately providing sweat samples for analysis.

One or more examples disclosed herein may provide the suggested uses and improvements mentioned above.

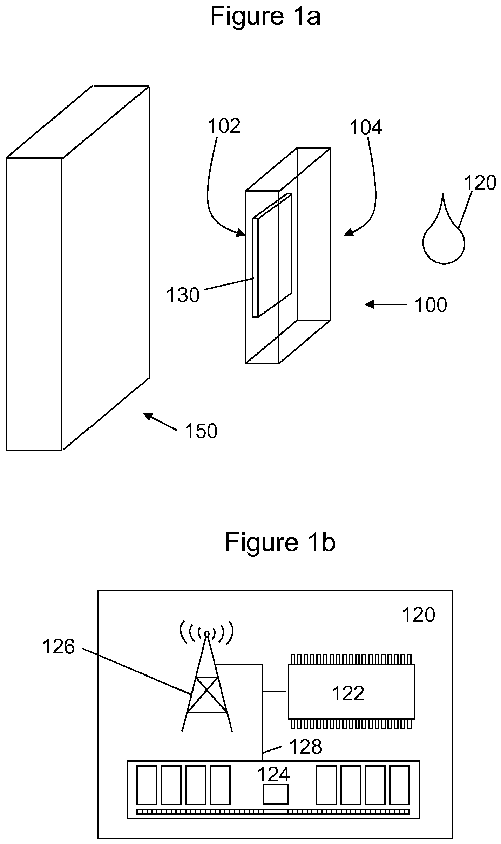

Figure 1a shows an example hand device 150, and hand device module 100 comprising a sweat detector 130 according to the present disclosure. The hand device 150 and hand device module 100 are shown as separate components in Figure 1a. In practice/use, the hand device module 100 would be affixed/attached to the hand device 150.

The hand device module 100 comprises an attachment face 102 and a sweat receptor face 104. The attachment face 102 is configured to be attached to the hand device 150. The sweat receptor face 104 is configured to receive sweat 120 when the module 100 is attached to the hand device 150. The module 100 is configured to provide the received sweat 120 to a sweat detector 130. The sweat detector 130 is configured to detect one or more properties of the received sweat 120. The sweat detector 130 may be a part of the hand device module 100 in some examples.

The module 100 may comprise means for providing the received sweat from the sweat receptor face to the sweat detector. For example, the module 100 may comprise one or more capillary channels configured to feed the received sweat from the sweat receptor face to the sweat detector. A capillary channel may be configured with a diameter of a size which acts to wick/draw the sweat through from the sweat receptor face to the sweat detector by capillary action. In some examples, the means for providing the received sweat to the sweat detector may comprise one or more of a pore (e.g. a single small hole through which sweat may pass but which is too small for the user's skin to directly contact the sweat detector), a valve (e.g. a hole through which sweat may pass from the sweat receptor face of the module to the sweat detector, but may not pass from the sweat detector to the sweat receptor face), an opening (e.g. a space in the module through which sweat may pass to the sweat detector and which may allow skin contact with the sweat detector) or a passage (e.g. a straight, or bent, pipe/tube through which sweat may travel but which may not necessarily draw the sweat liquid through by capillary action). In some examples the module may comprise a sponge/sponge-like porous material (e.g. an irregular network of conjoined pores) through which sweat may travel from the sweat receptor face to the sweat detector.

The hand device 150 is be a device which the user holds, or contacts, with their hand. By the user holding/contacting the hand device 150, and thereby holding/contacting the attached hand device module 100 with their hand, sweat 120 from the user's hand may be transferred from the sweat receptor face 104 of the hand device module 100 to the sweat detector 130 which can detect one or more properties of the received sweat 120. The sweat detector 130 may be located at the attachment face 102 of the hand device module 100, may be located at the sweat receptor face 104 of the hand device module 100, or may be located within the hand device module 100 between the attachment face 102 and the sweat receptor face 104.

The holding/contacting of the hand device 150 by the user may be on a regular basis. In this way, the hand device 150 and hand device module 100 may be used, with the sweat detector 130, to receive and analyse the user's sweat on a regular basis. If the user usually holds/contacts the hand device 150 anyway in daily use, then the user's sweat 120 may be received and analysed without the user being required to make a conscious decision to provide sweat for analysis. This may be termed "compliance free" sweat analysis because the user's sweat may be received and analysed without the user being required to change their usual behaviour/habits.

For example, the user may be a driver and the hand device 150 may be a steering wheel or gearstick. The hand device 150 may be a hand portable electronic device which the user regularly holds, such as a mobile telephone, smartphone, or music player. The hand device 150 may be a pen, electronic stylus or other writing implement and the user may regularly write letters or notes or draw on a graphics tablet, for example. The hand device 150 may be a handheld computer peripheral, such as a mouse, joystick, or trackball. The hand device 150 may be a handlebar of a bicycle for a user to hold when riding the bicycle. The hand device 150 may be the handset of a desktop telephone. The hand device 150 may be a handle of an object which the user regularly holds, such as of a bag or briefcase, or a banister or handrail in a bathroom (for example of an elderly or disabled person). The hand device 150 may be a tool, such as a hammer, chisel, screwdriver, plane or drill (and the person may be a joiner or carpenter and so hold the tool regularly).

The hand device module 100 may be termed a hand portable electronic device module 100, for example if it is attached to a portable electronic device such as a mobile telephone.

In some examples the hand device module 100 may be separate from and attachable to the hand device 150. In some examples the hand device module 100 may be separate from and attachable to the sweat detector 130. In some examples the hand device module may be replaceably attachable to the hand device 150. In some examples the hand device module may be replaceably attachable to the sweat detector 130. In some examples the hand device module 100 may be an integral part of the hand device 150. In some examples the hand device module 100 may be an integral part of the sweat detector 130.

The sweat detector 130 is shown as an integral part of the hand device module 100. In some examples the sweat detector 130 may be separate from, and attachable to (in some examples replaceably attachable to) the hand device module 100.

A repeatably attachable attachment face 102 of the hand device module 100 may be repeatably attached by one or more of an adhesive and a physical locking element. Similarly, a repeatably attachable sweat detector 130 may be repeatably attached by one or more of an adhesive and a physical locking element.

In the hand there may be slight differences in the composition of the sweat produced at the users' fingers and at the user's palms. During exercise and normal daily living any discrepancy between sweat composition from a user's fingers and a user's palms would be negligibly minor. Larger differences may be present during very profuse sweating caused by mental stress. In cases in which differences in sweat composition are present between different parts of the user's hand (e.g. the fingers or the palm) then the same portion of the user's hand is likely to contact the sweat detection face 104 of the hand device module 100 upon repeated contact from the user, thus any differences may be assumed to be due to a difference in composition of the sweat rather than a difference due to a different body part contacting the sweat detector though the user's physiology is the same.

Figure 1b shows an example apparatus 120 according to the present disclosure. Such an apparatus 120 may be, or may be a module of, a hand device such as hand device 150 onto which a sweat receptor 130 and hand device module 100 may be affixed. The apparatus 120 comprises a processor 122 and memory 124 (including computer program code) and a transceiver 126, which are electrically connected to one another by a data bus 128.

The processor 122 may be configured for general operation of the apparatus 120 by providing signalling to, and receiving signalling from, the other components to manage their operation. The memory 124 may be configured to store computer code configured to perform, control or enable operation of the apparatus 120. The memory 124 may also be configured to store settings for the other components (such as a camera or optical sensor configured to detect the effect of received sweat 120 on the sweat detector 130). The processor 122 may access the memory 124 to retrieve the component settings in order to manage the operation of the other components (e.g. a camera or optical sensor).

The transmitter 126 may comprise a separate transmitter and receiver and is configured to transmit data to, and receive data from, one or more other devices, such as a remote server, via a wireless or a wired connection. For example, if the apparatus 120 forms part of a hand device 150, the transceiver 126 may be configured to transmit the captured optical information (e.g. a colour change) indicating the effect of the received sweat 120 on a chemical composition present in the sweat detector 130 to a remote server for analysis. The transceiver 126 may, in some examples, receive a health indication from a remote server which has analysed the information from the sweat detector 130 so the health indication may be provided to the user via a user interface (e.g. display screen of the apparatus 120).

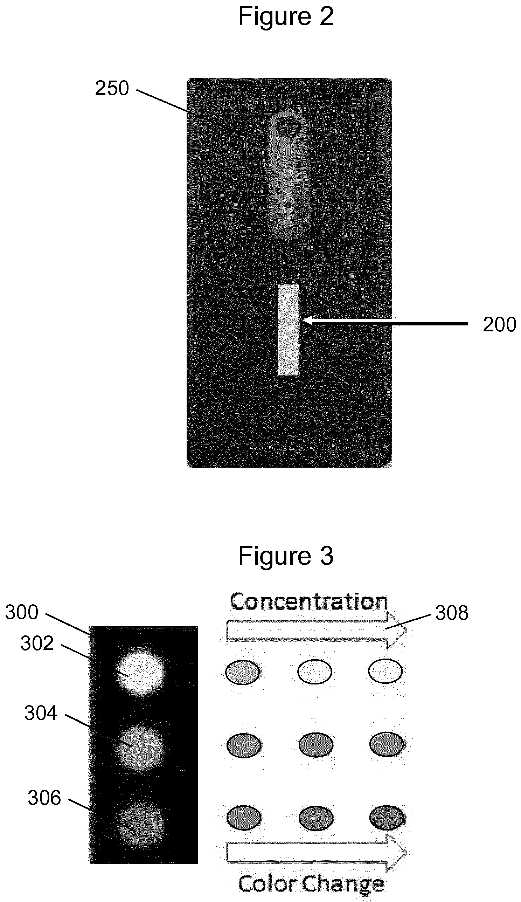

Figure 2 shows an example hand device 250 and hand device module 200 according to the present disclosure. In this example, the hand device 250 is a smartphone and the hand device module 200 is located on the back face of the smartphone 250 where the user's palm will make contact when the user is holding the smartphone 250 to interact with the front (e.g. touch screen) face or when holding it to their ear to make a telephone call. In this way sweat from the user's palm will reach the hand device module 200 when the user is using their smartphone 250.

The hand device module 200 in this example is configured so that the hand device 250 is attachable to the attachment face of the hand device module 200, so that when the hand device 250 is attached to the hand device module 200, the sweat receptor face of the hand device module 200 faces outwardly towards a user of the hand device 250.

In this example, the hand device module 200 is located on the back face of the smartphone 250. In other examples, the hand device module 200 may be located at the side/edge of the smartphone; for example, if the user's grip is such that the side/edge is more prone to receiving a user's sweat from a palm or fingers. A hand device 200 may have more than one attached (or integral) hand device module 250 for sweat analysis from more than one area of the user's hand.

The hand device in other examples may be, for example, another type of hand portable electronic device, such as a mobile telephone, a tablet computer, an electronic navigation device, a smartwatch, or a music player. The hand device may, in some examples, be a cover/case for such a hand portable electronic device. The case may be a protective case configured to provide protection for an encased device (for example, to reduce the chance of damage/breakage if the device is dropped, or reduce/prevent the device being scratched or chipped if e.g. it is placed in a pocket with something sharp such as a bunch of keys). In other examples, the hand device may be a writing implement, a steering wheel, a handlebar, a handle, a tool, a handheld computer peripheral, or a cover/sheath/module for the same.

Certain compounds which may be found in a user's sweat may be present "naturally", for example, as a biological analyte present as a component in most people's sweat (but different users' sweat may have different levels/ratios of such naturally occurring sweat compounds). Examples include water, sodium ions, chloride ions and potassium ions. Thus, certain examples disclosed herein may be used in the detection of, for example, medical conditions which result in an "imbalance", or presence, of one or more compounds in the user's sweat which would not otherwise be present at that level when the user is in a healthy state.

Certain compounds which may be found in a user's sweat may be present "unnaturally" or "artificially", for example, as an analyte present due to, for example the user consuming certain products, or consuming an extreme amount of a particular product (e.g. consuming prescribed and/or illegal drugs/medication, or consuming high levels of alcohol, sugar, or meat) which produces particular compounds excreted in the user's sweat. Other examples of "unnatural" sweat components include those present due to a medical condition which causes the production of a compound excreted in the user's sweat which users not suffering from the medical condition would not have present in their sweat.

Thus, examples disclosed herein may be used in the detection of, for example, illegal drug taking, the taking of drugs prohibited by certain activities (such as performance enhancing drugs taken by a sports cheat), the consumption of unhealthy levels of certain products which may be fine for consumption in moderation (e.g. alcohol, red meat, animal fats), or checking that a user is taking medication in a suitable dosage regime.

Figure 3 shows an example hand device module 300 comprising a plurality of chemical compositions 302, 304, 306 according to the present disclosure. The hand device module 300 comprises a sweat detector in this example.

The sweat detector is configured to detect one or more properties of the sweat by using one or more chemical compositions 302, 304, 306 which interact with the sweat according to the one or more properties of the sweat. In this example, the interaction of the one or more chemical compositions 302, 304, 306 with the sweat is a respective specific change in the colour of at least one of the one or more chemical compositions 302, 304, 306 according to the detected one or more properties of the sweat. Of course, while there are three example chemical compositions shown in Figure 3, in other examples there may be a different number (e.g. one, two, four, five, or more than five) chemical compositions. In some examples there may be a plurality of regions/chambers comprising the same chemical composition, so that the same sweat compound may be detected by more than one chemical composition region/chamber (e.g. to improve statistics/for quality control/as a check).

That is, different chemical compositions 302, 304, 306 are configured to react with different possible components of a user's sweat to each produce a colour change depending on the components present. By detecting if a colour change takes place (and in this example, by determining the extent of the colour change) the presence and concentration 308 of one or more reactive sweat components can be established. Such sweat components may be, for example, water, glucose, Na⁺, Cl⁻, and other biomarkers.

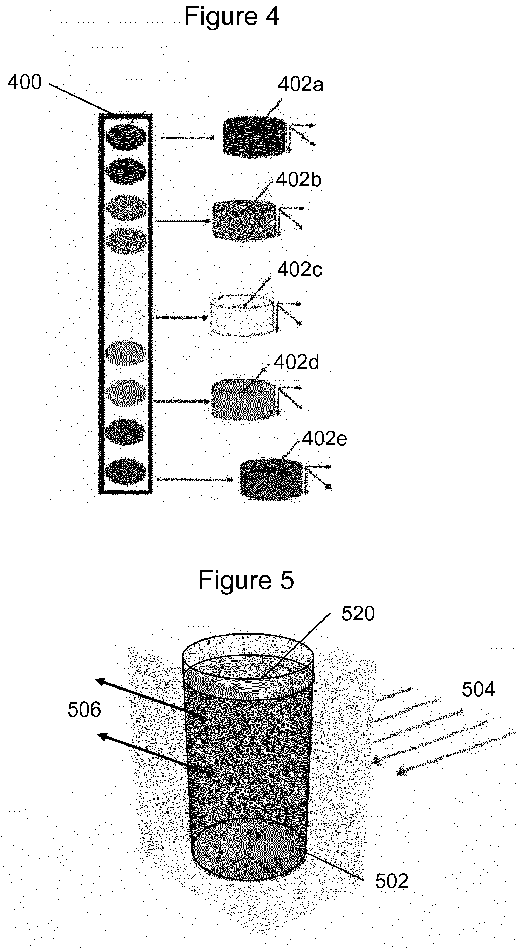

The hand device module 300 may comprise one or more chambers/reservoirs in which the sweat is captured (this is described in more detail with reference to Figure 4). The one or more chemical compositions 302, 304, 306 may be located, for example, at the bottom of respective miniature reservoirs. In examples where the hand device module 300 does not house the chemical compositions in chambers, they may be located on a flat patch region of the hand device module 300.

On contact with with specific compounds found in sweat, one or more of the particular compositions 302, 304, 306 may undergo a specific colour change. The hand device, or the hand device module 300, may comprise a camera configured to capture the colour changes. The colour changes can be analysed using an image analysis algorithm. which can statistically determine a health status prediction. (e.g. by comparing the results for the current user with an expected "theoretical" result and/or results from one or more comparable other users, e.g. from the same demographic group as the current user).The sweat detector 300 may be, in some examples configured to provide one or more properties of the sweat, by using the one or more chemical compositions 320, 304, 306 as described above, and providing one or more colour changes of the one or more chemical compositions to an analysis module (not shown). The analysis module is configured to provide a health status prediction to the user. The analysis module may, for example, be comprised in the hand device, or may be remote from the hand device module and the hand device. Communication of the detected colour changes may take place from the sweat detector, via the hand device, to the remote location of the analysis module, for analysis. In certain examples in which the hand device is a "dumb" (e.g. non-electronic) device, such as a hand tool or handle, communication to an analysis module may be made from the sweat detector via communication using a further electronic device capable of sending and receiving data signals, such as the user's smartphone or smart watch, for example. The analysis module may, in some examples, be a "virtual module" present in the "Cloud" or on the Internet. In some examples, it may be a physical server.

The analysis module may have access (e.g. to a database) of reference colours and health statuses associated with the colours, including the degrees of change in colour corresponding to different concentration levels of the analyte component of the sweat. The analysis module may also have access to calibration data for the user in a healthy state, so that the detected colour changes can also be compared to a "normal" healthy state for the particular user.

The analysis module may determine, for example, that the change in colour of a particular compound indicates that there is a higher than expected level of sodium ions in the user's sweat, and that this indicates the user is dehydrated. The analysis module may send a signal to a user device (for example, the hand device being used for sweat analysis, or another user device such as a smartwatch) to provide a health status message to the user, such as "you need to have a drink of water" or "you are dehydrated, have a rest and a drink". The health status may be provided in any suitable way, for example by a visual text based or image based message on a screen of a user device, as an audio alert (e.g. an alarm or a spoken/recited message in words), or as a haptic (e.g. vibration) indication to prompt the user to check their personal device for a health status update.

As examples of analytes present in sweat which may be detected by a colour changing chemical composition:
- water (analyte) causes CoCI2 (cobalt chloride) (for example, in a poly (2-hydroxyethyl methacrylate (PHEMA) support) to change colour from blue to purple.
- glucose oxidase produces hydrogen peroxide by the oxidation of glucose (analyte) and reduction of oxygen. Peroxidase then oxidises iodide (chemical composition) to form iodine, showing a colour change of yellow (iodide) to brown (iodine).
- chloride (analyte) reacts with Hg²⁺ and Fe²⁺ with 2,4,6-tris(2-pyridiyl)-s-triazine (TPTZ) (chemical composition). Hg²⁺ and Fe²⁺ competitively try to bind to 2,4,6-tris(2-pyridiyl)-s-triazine (TPTZ). However, in the presence of chloride ions, iron ions (Fe²⁺) bind with TPTZ because Hg²⁺ binds with chloride ions to form HgCl₂, thereby causing a transparent to blue colour change. The Hg²⁺ would be present in non-toxic/low toxicity levels and/or as part of a non-toxic/low toxicity compound (e.g. thiomersal/ ethylmercury).
- pH and Na⁺ (sodium ion)) (analyte) concentration are correlated and may be detected using bromothymol blue (bromothymol sulfone phthalein, or BTB) methyl red ((2-(N,N-dimethyl-4-aminophenyl) azobenzenecarboxylic acid), and/or phenolphthalein (chemical compositions). Bromothymol blue is a pH indicator which is yellow below a pH of 6.0, green/blue around neutral pH 7, and a deep blue above a pH of around 7.6. Methyl red is a pH indicator which is red below a pH of 4.4 and yellow above a pH of 6.2, with an orange tone between pH=4.4 and 6.2. Phenolphthalein is a pH indicator which is colourless below a pH of around pH = 8.2 and pink/fuchsia above a pH=8.2.

Further, in some examples, the chemical composition need not be in direct contact with the user's hand/skin when the user is holding the hand device module/hand device. For example, a thin transparent protective inert film/membrane/layer may be present over the chemical composition (e.g. in the chamber) to act as a "barrier" between the chemical composition and the user's skin which prevents/reduces direct contact between the chemical composition and skin but allows the passage of sweat through the layer. Once sweat droplets from the user's skin have been produced, the sweat can pass through the layer to the chemical composition for reaction therewith. The inert protective layer acts to prevent direct contact between the user's skin and the chemical composition(s).

Examples of what analytes/compounds may be present in sweat, and what conditions/disorders their presence in sweat may indicate, include:
Na⁺ - Cystic fibrosis, dehydration, and endocrine diseases such as diabetes
Cl⁻ - Cystic fibrosis, dehydration, adrenal disease, thyroid disease, and pituitary glands disease
K⁺ - Blood pressure disorders, muscle contractions, nerve impulses, digestion disorders, and heart rhythm problems
NH₄⁺ - Liver problem, kidney problem, and electrolyte imbalance
Ethanol - Intoxication e.g.by alcohol consumption
Cortisol - Stress
Urea - Kidney problems
Lactate - High level of anaerobic activity
Neuropeptides and Cytokines - Depressive/personality disorders

Figure 4 shows an example hand device module 400 comprising a plurality of chambers 402a - 402e containing respective chemical compositions according to the present disclosure. The hand device module 400 may be a standalone apparatus configured for use with a hand device, or may be integral to a hand device.

Again, as in Figure 3, the sweat detector is configured to detect one or more properties of the sweat by using one or more chemical compositions 402a-402e which interact with the sweat according to the one or more properties of the sweat. The interaction of the one or more chemical compositions with the sweat is a respective specific change in the colour of at least one of the one or more chemical compositions 402a-402e according to the detected one or more properties of the sweat. Figure 4 shows that the hand device module 400 comprises a plurality of chambers, and the one or more chemical compositions 402a-402e are comprised in the one or more respective chambers of the module 400.

In an example of the hand device module 400 being separate from and configured for use with a hand device, the hand device itself may comprise one or more chambers configured to receive the received sweat from the sweat receptor. A hand device module 400 may comprises corresponding chambers configured to sit/be positioned inside the chambers of the hand device and if, e.g. colour-changing chemical compositions are to be used for sweat analysis, these compositions are located in the chambers of the hand device module 400.

The sweat detector in some examples, and the hand device, in other examples, may comprise one or more cameras configured to capture one or more changes in colour, due to interaction with the sweat, of the one or more chemical compositions 402a-402e in the one or more chambers. In some examples there may be one camera per chamber. In this case the processing may be simple, to take one colour change captured by one corresponding camera and determine the presence of a particular analyte in the sweat from the captured colour change. In some examples there may be one camera per a plurality of chambers. In this case, the camera apparatus may be simpler because fewer cameras are required than if using one camera per chamber. However, the image captured by the camera may need to be segmented according to the different regions of colour changing chemical composition present in the chamber, to determine the presence of one or more particular analytes in the sweat from the captured colour changes. In some examples there may be more than one chemical composition per chamber. The chamber may be imaged by one or a plurality of cameras.

Figure 5 shows an example chamber 502 configured to allow the refraction property of the sweat 520 within the chamber 502 to be determined. One or more light sources 504 (e.g. white LEDs) may be present for providing light to be directed into the sweat in the chamber 520. A refraction sensor may be used to capture the refracted light 506 and analyze the refraction capability of the sweat liquid 520 contained in the miniature chamber/reservoir 502. A refractometer (not shown) may be used to determine the refractive index of the sweat 520. A refraction sensor, and/or a refractometer, may be present as part of a sweat detector, or as part of a hand device.

Light 504 scatters differently depending on the type and concentration of dissolved material in the sweat 520. Therefore, the refractive index of the sweat 520 may be determined from the concentration of the dissolved material. For example, sweat with a high concentration of chloride ions Cl⁻ will have a different refraction capability than sweat with high amounts of both sodium, Na⁺, and chloride, Cl⁻, ions.

In theory, all analytes may affect the refractive index of sweat at different concentrations, and the detection of analytes in sweat by refractive analysis is not limited to the detection of sodium and/or chloride ions. The exact correlations between analyte presence and concentrations and refractive index may be established, for example through calibration measurements.

Thus, the sweat detector may be configured to detect the one or more properties of the sweat, wherein the one or more properties of the sweat comprises a refraction property of the sweat. A relationship between the refractive index of sweat liquid and the concentration of one or more compounds in the sweat may be predetermined in a calibration stage. Then, in use, refraction results may be analysed to determine the presence of one or more particular materials in the sweat. In some examples, lights 504 of different wavelengths may be used to detect each different compound or a plurality of compounds in the sweat. The light source(s) may be, for example, single colour LEDs and/or one or more variable colour LEDs (e.g. RGB(W)). Light sources which are not LEDs may also be used (e.g. a bulb) although LEDs may provide a convenient source of light for inclusion in an electronic device system.

The chamber 502 of Figure 5 may be present in a hand device module, or in a hand device, as described above, and may receive sweat from a user. The chamber 502 (one is shown in Figure 5 but there may be a plurality of such chambers) comprises access to an optical sensor configured to determine a refraction property of the sweat within the chamber. The sweat detector, or the hand device, may also therefore comprise one or more optical sensors configured to capture a refraction property of the sweat. The chamber 520 may have transparent walls, or walls with a portion of transparent material, to allow the passage of light through the chamber walls into and out of the sweat contained within, thereby providing access to the optical sensor for refracted light from the sweat 520.

In some examples, if both chemical compositions such as those described in relation to Figures 3 and 4, are used as well as a refraction measurement as described in relation to Figure 5, the chemical composition technique may determine one or more properties of the sweat while the refraction technique may identify another different property of the sweat. In some examples, one chamber may comprise one or more chemical compositions, for example at the base of the chamber, configured to change colour upon reaction with an analyte in a user's sweat, and the same chamber may be configured such that light is provided into the chamber and is refracted, and the refracted light is captured by an optical sensor (for example, the light source and optical sensor may be arranged around the periphery of the side walls of the chamber).

The hand device module may be removeable/replaceable to allow for the module to be refreshed following use. For example, if the module comprises chemical compositions configured to change colour upon reaction with sweat, it may be that, after a number of uses (e.g. 5 or 10), the chemical composition has been "used up" and new chemical composition material needs to be used for further sweat analysis. As another example, if the module comprises a chamber configured to capture sweat for refractivity analysis, it may be beneficial to remove the module for cleaning (e.g. rinsing with clean water) regularly (e.g. once a week or month) to keep the chamber clean and remove residual matter from dried sweat and/or contaminants (e.g. dust).

In some examples, it may be desirable to trigger the activity of, for example, a camera configured to capture the change in colour of a colour change chemical composition, or the optical sensor/detector configured to detect the refraction properties of sweat in a chamber. In this way, the camera/optical sensor need not continuously capture optical information, but may capture optical information only when a change is detected, for example.

In some examples the optical sensor(s) may be triggered to capture optical information from the chamber(s) upon a user-initiated instruction (for example, provided by a user to an application running on the hand device). In some examples the optical sensor(s) may be triggered to capture optical information from the chamber(s) upon a user-initiated input which is not a conscious "capture" input made by the user but is detected based on the user handling the hand device. For example, a pressure or heat sensor may detect that a user has picked up/held the hand device and this detected grip is used to trigger the capture of optical information from the one or more chambers of the hand device module. As another example, a light sensor may be present proximal to the chamber(s). Upon detecting that the light sensor has been covered (by the user holding the hand device) the capture of optical information from the chamber is triggered. There may be a predetermined time delay between detection of a user hold/grip on the hand device and triggering optical information capture, to allow time for sweat to be produced and cause a detectable effect (e.g. colour change or filling a chamber).

One or more such detection methods may be used. For example, to avoid triggering optical information capture by the user simply placing a hand device down on a surface and covering a light sensor, both information from a light sensor indicating the chamber/sensor have been covered, and information from a heat sensor detecting that the user has made skin contact with the hand device may be required to trigger a measurement. As another example, a light sensor may be used to determine that the chamber has been covered, as well as an indication form the hand device indicating that the device is in use (for example, an application has been opened, or the device has been "unlocked" for use by a user entering a password).

In some examples, there may be one or more shutters present on the hand device module. A shutter may be configured to cover the opening to a corresponding chamber until a trigger signal is received, indicating that a user has placed a hand/body part over the chamber/shutter, upon which the shutter is configured to open to allow sweat to enter the chamber. The sensor opening may be triggered in a similar way to that described above in relation to capturing optical information (e.g. upon detecting a heat/pressure/light sensor covering/device in-use signal). By using a shutter, the entrance of dust, fluff and other contaminants from the environment into the chamber may be reduced compared to a chamber which is always open to the environment.

In some such examples, the shutter may comprise, on the chamber side, a cleaning element configured to remove contaminants from the chambers. For example, a small brush may be present such that, when the shutter is moved back and forth to close/open the chamber, the brush acts to sweep away any dust or dirt which may have entered the chamber. In some examples, the shutter may comprise an absorbent cleaning element to remove sweat from the chamber following capture and analysis of the sweat. Thus, when the shutter closes following the receipt of a user's sweat, the absorbent cleaning element can wick away/absorb residual sweat so that the chamber is dry for subsequent sweat capture and analysis.

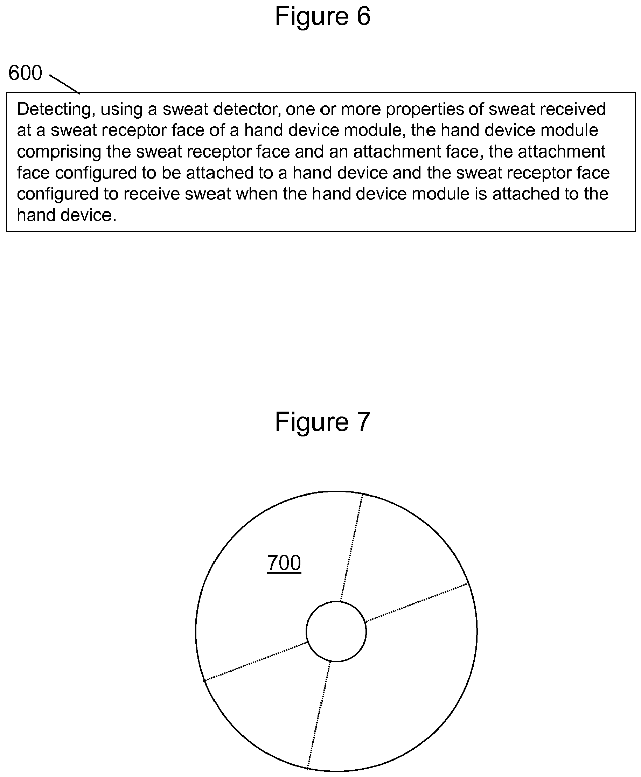

Figure 6 shows the main steps of a computer-implemented method of using the present apparatus. The method 600 comprises detecting, using a sweat detector, one or more properties of sweat received at a sweat receptor face of a hand device module, the hand device module comprising the sweat receptor face and an attachment face, the attachment face configured to be attached to a hand device and the sweat receptor face configured to receive sweat when the hand device module is attached to the hand device.

Figure 7 shows an example computer-readable medium comprising a computer program configured to perform, control or enable the method of Figure 6 or any method described herein. The computer program may comprise computer code configured to perform the method(s). In this example, the computer/processor readable medium 700 is a disc such as a digital versatile disc (DVD) or a compact disc (CD). In other examples, the computer/processor readable medium 700 may be any medium that has been programmed in such a way as to carry out an inventive function. The computer/processor readable medium 700 may be a removable memory device such as a memory stick or memory card (SD, mini SD, micro SD or nano SD card).

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some examples, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such examples can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc.), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed examples may consist of any such individual feature or combination of features. In view of the foregoing description it will be evident to a person skilled in the art that various modifications may be made within the scope of the disclosure.

While there have been shown and described and pointed out fundamental novel features as applied to different examples thereof, it will be understood that various omissions and substitutions and changes in the form and details of the devices and methods described may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or example may be incorporated in any other disclosed or described or suggested form or example as a general matter of design choice. Furthermore, in the claims means-plus-function clauses are intended to cover the structures described herein as performing the recited function and not only structural equivalents, but also equivalent structures. Thus, although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. A hand device module, the module comprising:
an attachment face and a sweat receptor face, the attachment face configured to be attached to a hand device and the sweat receptor face configured to receive sweat when the module is attached to the hand device, the module configured to provide the received sweat to a sweat detector, the sweat detector configured to detect one or more properties of the received sweat.

2. The module of claim 1, wherein the module is a hand portable electronic device module.

3. The module of claim 1 or claim 2, wherein the sweat detector is integrated with the module and the sweat detector is configured to detect the one or more properties of the sweat, wherein the one or more properties of the sweat comprises a refraction property of the sweat.

4. The module of any preceding claim, wherein the sweat detector is integrated with the module and the sweat detector is configured to detect one or more properties of the sweat by using one or more chemical compositions which interact with the sweat according to the one or more properties of the sweat.

5. The module of claim 4, wherein the interaction of the one or more chemical compositions with the sweat is a respective specific change in the colour of at least one of the one or more chemical compositions according to the detected one or more properties of the sweat.

6. The module of claim 5, wherein the module comprises one or more chambers and the one or more chemical compositions are comprised in the one or more respective chambers of the module.

7. The module of any preceding claim, wherein the module is configured to transfer the received sweat to a sweat detector integrated with the hand device.

8. The module of any preceding claim, configured so that the hand device is attachable to the attachment face of the hand device module, so that when the hand device is attached to the hand device module, the sweat receptor face of the hand device module faces outwardly towards a user of the hand device.

9. The module of any preceding claim, comprising means for providing the received sweat from the sweat receptor face to the sweat detector, the means comprising one or more of a:
a capillary channel;
a pore;
a valve;
an opening; and
a sponge.

10. A hand device comprising a sweat receptor, the sweat receptor positioned for engagement with one or more of the palm(s) and digit(s) of a user's hand(s) when the hand device is being held in one or more designed operative in-use holding positions, the sweat receptor configured to be operatively connected to a sweat detector of the hand device.

11. The hand device of claim 9 or claim 10, wherein the hand device comprises one or more chambers configured to receive the received sweat from the sweat receptor, the one or more chambers comprising one or more of:
one or more chemical compositions configured to interact with the sweat according to the one or more properties of the received sweat; and
access to an optical sensor configured to determine a refraction property of the sweat within the chamber.

12. The hand device of any of claims 9 to 11, wherein the sweat detector comprises one or more of:
one or more cameras configured to capture one or more changes in colour, due to interaction with the sweat, of the one or more chemical compositions in the one or more chambers; and
one or more optical sensors configured to capture a refraction property of the sweat.

13. The hand device of any of claims 9 to 12, wherein the sweat detector is configured to provide the one of more of the detected refraction property of the sweat; and the one or more properties of the sweat by using one or more chemical compositions which interact with the sweat according to the one more properties of the sweat, to an analysis module configured to providing a health status prediction to the user.

14. A method comprising:
detecting, using a sweat detector, one or more properties of sweat received at a sweat receptor face of a hand device module, the hand device module comprising the sweat receptor face and an attachment face, the attachment face configured to be attached to a hand device and the sweat receptor face configured to receive sweat when the hand device module is attached to the hand device.

15. A computer readable medium comprising computer program code stored thereon, the computer readable medium and computer program code being configured to, when run on at least one processor, perform:
detecting, using a sweat detector, one or more properties of sweat received at a sweat receptor face of a hand device module, the hand device module comprising the sweat receptor face and an attachment face, the attachment face configured to be attached to a hand device and the sweat receptor face configured to receive sweat when the hand device module is attached to the hand device.
